# EUROPEAN PATENT APPLICATION

(11) **EP 4 806 453 A1**
(43) Date of publication of application: **16.09.2026**
(21) Application number: 25163886.2
(22) Date of filing: 14.03.2025
(51) Int. Cl.: A61K 38/17, A61K 9/00, A61K 31/7105, A61K 31/712, A61P 27/10

(54) **TARGETING OPSIN EXPRESSION WITH MRNA THERAPY FOR MYOPIA CONTROL AND MYOPIA TREATMENT**

(71) Applicant: Carl Zeiss Vision International GmbH, 73430 Aalen (DE)
(72) Inventor: GISBERT MARTINEZ, Sandra, 73430 Aalen (DE); WAHL, Siegfried, 73430 Aalen (DE)
(74) Representative: PATERIS PartmbB

(57) **Abstract**

A synthetic mRNA molecule is provided comprising a sequence encoding an opsin protein for use in a therapeutic method for preventing and/or treating myopia. It was surprisingly found that a synthetic mRNA molecule is suitable for the treatment of myopia. Further, the synthetic mRNA molecule is provided for use in a therapeutic method to increase intracellular opsin expression. Further, a use of the synthetic mRNA molecule is provided for the manufacture of a pharmaceutical composition for treatment and/or prophylaxis of development of myopia. Further, a pharmaceutical formulation is provided containing the synthetic mRNA molecule.

Moreover, a method is provided for manufacturing a pharmaceutical formulation comprising a mRNA molecule carrying the coding information for an opsin protein, the method comprising the steps of:
- S1: Selecting a target opsin protein whole expression needs to be increased,
- S2: Providing DNA encoding the amino sequence of the target opsin protein within an expression vector,
- S3: Producing mRNA by *in vitro* transcription,
- S4: Mixing the synthesized mRNA with at least one delivery agent,
- S5: Formulating the mixture of the synthesized mRNA and the delivery agent into a pharmaceutical composition.

## Description

The present invention relates to polynucleotide sequences carrying the coding information for a protein related to the control of eye growth. In particular, the invention relates to a mRNA fragment carrying the coding information of an opsin protein for use as a therapeutic tool in the treatment and control of myopia. In addition, the invention relates to a pharmaceutic formulation comprising the mRNA fragment. Furthermore, the invention relates to a method for preparation of the mRNA fragment and the pharmaceutical formulation.

Myopia, also known as short-sightedness, is an eye-condition where light from distant objects focuses in front of, instead of on, the retina. The main cause of myopia is due to excessive ocular elongation, which creates a mismatch between the optical power and the length of the eye, resulting in blurred vision at far distances. This refractive error can be corrected using eyeglasses or contact lenses which are the primary choices for correction. Additionally, there are other alternatives such as orthokeratology, refractive surgery procedures, or vision therapy. However, these options do not address the underlying cause of myopia development; instead, they correct the refractive issue particularly by focusing light rays on the retina, allowing individuals to achieve good visual performance.

In the pursuit of understanding the mechanisms underlying myopia progression, several studies have indicated that cone photoreceptor function may play a key role. The study by Gisbert et al. (2018) reported a correlation between M to L cone ratios and the length of vitreous chamber depths and refractive states in control eyes with normal vision in the chicken retina. These findings showed that a higher proportion of L cones relative to M cones was associated with deeper vitreous chambers and less hyperopic refractive states. Furthermore, it was found that M to L cone ratios were also correlated with the amount of deprivation myopia induced, but these correlations were inverted compared to those in fellow control eyes (Gisbert et al., 2022). In humans, an association between symmetric L to M cone ratios and a higher susceptibility to myopia development has been proposed (Neitz & Neitz, 2015). This is supported by the fact that individuals with red-green color vision deficiencies, which have an unbalanced propulsion of L and M cones due to the absence of one of them, exhibit highly skewed ratios and present lower rates of myopia (Ostadimoghaddam et al., 2014). These findings suggest strong evidence for a cone based driven emmetropization response, which is the visual guided process that adjusts the axial length of the eye to match the power of its optical components.

Cone photoreceptors play a fundamental role in the process of vision. Their importance lies in their ability to convert light into electrical signals, through a process called phototransduction, which are then transmitted to the brain. In this process, opsin photopigments, found in the outer segments of cones, are crucial molecules. Each type of cone contains a specific type of opsin that responds to different wavelengths of light. These molecules initiate a cascade of biochemical reactions, generating electrical signals that are subsequently transmitted to downstream neurons. Several studies have explored the impact of opsin expression on myopia. Ji et al. (2021) investigated the role of an opsin in emmetropization using a mouse model. The induction of a mutation affecting the expression of M-opsin, to make mice less responsive to greenlight, impacted the refractive development. The results show that wild type mice developed a myopic shift in eyes exposed to green night, while mice carrying the mutation developed more hyperopic refractions. A recent study found that mRNA red opsin expression was reduced in chickens after inducing myopia either with diffusers or negative lenses (Gisbert et al., 2023). Given the outcomes, changes in opsin expression may therefore alter the photoreceptor output and consequently the signal transmitted to downstream retinal neurons.

Furthermore, genetic studies in humans have identified haplotype variants in exon 3 of the L and M opsin genes (OPN1LW and OPN1MW, respectively) that are associated with some forms of myopia (McClements et al., 2013; Neitz et al., 2021). These variations lead to a significant decrease in the opsin content in cone photoreceptors carrying the mutation. The resulting cone arrangement in the retina, formed by cones expressing normal amounts of photopigment and cones nearly devoid of photopigment, may cause errors in contrast signalling promoting eye growth (Greenwald et al., 2017; Hagen et al., 2019; Neitz et al., 2021).

Conventional approaches to treat myopia mentioned above primarily address the symptoms rather that the biological causes of the condition. Furthermore, these approaches are not stopping the progression, but only slowing it down, and on top there are still a significant number of non-responders. Several myopia control strategies that aim to slow down myopia progression are also available. The use of control myopia lenses, atropine eye drops, orthokeratology and red-light therapy have been found to modulate myopia progression. However, these treatments may not be accessible to everyone due to cost and availability. Furthermore, approaches comprising the application of myopia control lenses, contact lenses, and orthokeratology may be challenging to handle, particularly for children.

It is an object of the present invention to provide an effective means for addressing the biological cause of myopia.

The object of the present invention is solved by the teaching of the independent claims. Further advantageous embodiments and aspects of the invention are evident from the dependent claims, the description, the figures and examples. The embodiments of the invention can be combined in a beneficial way.

Messenger RNA (mRNA) is a single-stranded molecule of RNA that corresponds to the genetic sequence of a gene, from which it is transcribed. In a process called translation, which takes place in a ribosome, the mRNA is translated into a sequence of amino acids of a protein. Genomic sequences, e.g. introns, are removed from the mRNA prior to translation in a process called splicing. The structure of a mature eukaryotic mRNA comprises a 5'cap, a 5'UTR, a coding region, a 3'UTR, and a poly(A) tail. The 5'cap consists of a modified form of guanosine, namely 7-Methylguanosin. The 5'cap protects the mRNA from degradation by enzymes and facilitates the binding of the cap-binding process as well as the recognition of the mRNA by the small subunit of the ribosome. The 5'UTR (UTR = untranslated region) comprises regulatory sequences, binding positions for regulatory proteins and other molecules, and sequences for initiating the translation. The coding region provides the nucleotide sequence which is translated into an amino acid sequence of a protein by the ribosome. The 3'UTR comprises regulatory sequences, binding positions for regulatory proteins and a polyadenylation sequence followed by a number of adenine residues. When transcribed from genomic DNA, the mRNA contains introns which are removed by a process called splicing. When transcribed from cDNA or a synthesized DNA, mRNA does not contain introns. Synthetic mRNA can be transcribed *in vitro* from a DNA template.

Complementary DNA (cDNA) is a form of DNA which is derived from mRNA or a microRNA by reverse transcription. In natural forms, this process is carried out by retroviruses, where the cDNA is then integrated into a host's genome. This process can be carried out *in vitro.* cDNA can also be synthesized chemically. A cDNA can be inserted into a plasmid, which can be transferred into bacterial cells for duplication. That way sufficient amounts of DNA for producing mRNA can be provided. This process can also be used for the creation of cDNA libraries, which are useful for providing selected cDNAs.

A first aspect of the invention is directed to a synthetic mRNA molecule comprising a sequence encoding an opsin protein for use in a therapeutic method to increase intracellular opsin expression. It was surprisingly found that a synthetic mRNA molecule is suitable for the treatment of myopia. In contrast to direct protein delivery, mRNA provides sustained expression of therapeutic proteins, offering prolonged clinical benefits. Furthermore, transfection of mRNA is advantageous over traditional gene therapies, as mRNA is transiently expressed and does not integrate into the genome of a transfected cell, thereby enhancing overall safety of the treatment. The therapeutic use of mRNA enables the production of an individualized therapy compared to other myopia treatments and offers potential for long-term benefits by treating the underlying cause of myopia condition. The therapeutic use of mRNA has also the potential to function as a form of vaccination or preventive measure for myopia and its management, especially in early stages or pre-myopia.

Furthermore, the invention provides an advantageous approach because it enhances the expression of proteins involved in the biochemical pathways controlling eye growth. Consequently, the invention addresses the fundamental biological causes rather than the symptoms of myopia by enhancing the expression of opsin, thereby improving the insufficient production of opsin photopigment in the outer segments of cone photoreceptors. The invention is further advantageous because it aims to ensure proper eye growth and prevent the rapid increase in myopia that typically begins in school-age children and continues until late teenage years, significantly reducing the risk of sever ocular pathologies associated with high myopia, such as glaucoma, retinal detachment, macular degeneration, including blindness.

The encoded opsin protein is preferably at least one selected from the group comprising the human red opsin protein, the human green opsin protein, and the human blue opsin protein. The human red opsin protein is sensitive to long-wavelength light and is encoded by the gene OPN1LW. The human green opsin protein is sensitive to medium-wavelength light and is encoded by the gene OPN1MW. The human blue opsin protein is sensitive to short-wavelength light and is encoded by the gene OPN1SW. The application of a certain opsin mRNA could beneficially be related to an identified genetic cause, namely the above-mentioned haplotype variants, which result in a decrease in the opsin content in cone photoreceptors carrying the mutation. Cone photoreceptors carrying the malfunctioning haplotypes could be treated with e.g. red or green opsin mRNA, respectively, in order to provide normally functioning opsin proteins.

In the invention the sequence of the synthetic mRNA molecule is beneficially provided within an expression vector. The corresponding cDNA sequence comprises an open reading frame for the protein, a 5'cap, a 5'UTR, a 3'UTR, and a 3'poly A tail. In this approach, the information is provided for translation and processing of the mRNA into a functional protein, advantageously increasing the expression of an opsin protein into the transfected cells, namely in transfected cone photoreceptors. The information is sufficient for a transient expression, which is also advantageous in terms of regulation and safety. In an alternative embodiment, RNA can be used which comprises parts for self-amplification, namely sequences of a viral replication machinery.

Preferably the synthetic mRNA comprises an open reading frame (ORF) comprising at least one of a nucleotide sequence having at least 90%, 95%, or 100% identity with the nucleotide sequence of the coding sequence of the human red, green, or blue opsin protein, respectively. This is advantageous because the therapeutic use of mRNA offers the potential of an individualized therapy; sequences between individuals can vary.

A second aspect of the invention is directed to the use of a synthetic mRNA comprising a sequence comprising an open reading frame comprising at least one of a nucleotide sequence having at least 90%, 95%, or 100% identity with the nucleotide sequence of the coding sequence of the human red, green, or blue opsin protein for the manufacture of a pharmaceutical composition for treatment and/or prophylaxis of development of myopia. It should be clear that the synthetic mRNA to be used comprises at least one molecule of the cDNA of interest, but that in practice many copies of this mRNA molecule are used. Therefore, the term mRNA is used in the aspects of use and pharmaceutical applications below.

A third aspect of the invention is directed to a pharmaceutical formulation containing a synthetic mRNA comprising an ORF for an opsin protein, particularly for a human red opsin protein together with at least one pharmaceutically acceptable carrier, cryoprotectant, lyoprotectant, excipient and/or diluent.

A pharmaceutically acceptable carrier is a carrier suitable for preparation of a pharmaceutical composition that is acceptable for veterinary and/or human pharmaceutical use. Exemplary carriers are lactose, starch, sucrose, cellulose, magnesium stearate, dicalcium phosphate, calcium sulfate, talc, mannitol, ethyl alcohol.

A cryoprotectant in the context of the present application is a substance suitable to preserve the structure and function of nucleic acids and their delivery agents, such as lipid nanoparticles, under low temperatures and freezing processes. Exemplary cryoprotectants benefitial in the present application are amino acids, glycols, and saccharides.

A lyoprotectant in the context of the present application is a substance suitable to preserve the structure and function of nucleic acids and their delivery agents, such as lipid nanoparticles, upon a lyophilisation process (freeze-drying). Exemplary lyoprotectants benefitial in the present application are sucrose, trehalose, and mannitol.

Excipients are substances usually added to pharmaceutical compositions alongside the active ingredient in order to enhance the active ingredient's properties, improve long-term stabilization and others. In the context of lipid nanoparticles for nucleic acid delivery novel lipids can be used as functional excipients to modulate the biodistribution, pharmacokinetics, pharmacodynamics and efficacy of the nucleic acid.

Diluents are inactive ingredients that are added to pharmaceutical compositions that usually make up the major portion of the composition or dosage form. Suitable diluents include sugars such as lactose, sucrose, mannitol, sorbitol, and starches.

In a preferred embodiment, the pharmaceutical formulation contains an additional synthetic mRNA providing a coding information for at least one additional biomolecule selected from the group comprising scleral collagen, nyctalopin, glucagon, somatostatin, neurotensin, nitric oxide synthase enzymes, transforming growth factor betas and basic fibroblast growth factor. This is advantageous because the expression of at least one additional biomolecule simultaneously can provide a synergistic effect in the treatment of myopia progression.

In the pharmaceutical formulation, the mRNA is preferably formulated with at least one delivery agent selected from the group comprising lipid nanoparticles, lipids, polymers, peptides, virus-like particles, and cationic emulsion. It is particularly preferred that the synthetic mRNA is used for the preparation of lipid nanoparticles encapsulating the molecule. Lipid nanoparticles (LNP) are a subclass of lipid-based nanoparticles. LNPs provide an efficient delivery vehicle for RNA molecules, particularly mRNA. LNPs comprise phospholipids, cholesterols, ionizable lipids, and polyethylene glycol-derived lipids (PEGylated lipids). The mRNA molecules are encapsulated in the interior core though electrostatic interactions, particularly with the ionizable lipids. Polyethylene glycol-derived lipids stabilize the LNPs, particularly by decreasing recognition by mononuclear phagocyte system. Phospholipids and Cholesterols have a stabilizing effect on the structure of the LNPs. Being a fragile molecule which is easily enzymatically degraded, mRNA within LNPs is stabilized and protected from enzymatic degradation. The lipids of the LNPs also enhance the cellular uptake of the mRNA by the target cells. The targeting of the LNPs can be improved by providing surface molecules on the LNPs binding to selected molecules on the surface of the target cells, namely of cone photoreceptor cells.

The pharmaceutical formulation is preferably suitable for intravitreal injection. Intravitreal injection is the most suitable administration route in the treatment of myopia to ensure that the mRNA reaches the target cells. Further advantageous administration routes include subretinal injection and topical eye drops. Consequently, the pharmaceutical formulation is preferably also suitable for subretinal injection and topical eye drops. These methods advantageously deliver the product close to the target cells and are likely to enhance cellular uptake.

A fourth aspect of the invention is directed to the use of a synthetic mRNA molecule comprising a sequence carrying the coding information for an opsin protein and/or an inventive pharmaceutical formulation for use as medicament.

A fifth aspect of the invention is related to a method for manufacturing a pharmaceutical formulation comprising a synthetic mRNA carrying the coding information for an opsin protein, the method comprising the steps of:
- Selecting a target opsin protein whose expression needs to be increased,
- Providing DNA encoding the amino sequence of the target opsin protein within an expression vector,
- Producing mRNA by *in vitro* transcription,
- Mixing the synthesized mRNA with at least one delivery agent,
- Formulating the mixture of the synthesized mRNA and the delivery agent in a pharmaceutical composition.

The selection of a target opsin protein is directed to the selection of a specific type of an opsin photopigment, which has been shown to be less expressed in developing myopia. Increasing the expression of an opsin mRNA to normal levels is expected to be beneficial.

Preferably the delivery agent is a lipid nanoparticle. The benefits of lipid nanoparticles are mentioned above.

In a preferred embodiment of the method at least one selected from the group comprising the human red opsin protein, the human green opsin protein, and the human blue opsin protein as found in cones of the human eye is selected as a target protein.

The expression of red opsin protein has been shown to be down-regulated in the development of myopia. Preferably the DNA template comprises an ORF with the sequence of the coding sequence of the human red, green or blue opsin protein.

An eighth aspect of the invention is directed to a method for prevention and/or treatment of myopia, comprising administering to at least one eye of a subject an effective amount of a synthetic mRNA molecule comprising a sequence carrying the coding information for an opsin protein or the inventive pharmaceutical formulation.

The invention is explained in more detail on the basis of the figures and the examples.
- Figure 1: shows a flow chart of a method according to the invention.
- Figure 2: shows a schematic representation of a production of a mRNA pharmaceutical composition.
- Figure 3: shows a schematic representation of an application of the mRNA pharmaceutical composition to the human eye.

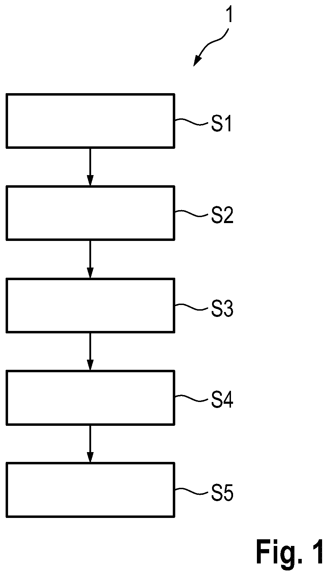

In Fig. 1 a method 1 is described for manufacturing a pharmaceutical formulation comprising a mRNA molecule carrying the coding information for an opsin protein. In a first step S1 a target opsin protein is selected and the DNA provided. The target opsin protein is an opsin which is related to myopia, whereby it is known to contribute to development of myopia by way of malfunction or not sufficient expression. Consequently, its expression needs to be increased. The red opsin protein of the human eye is here identified and selected as a target protein.

RNA samples from human retina are obtained from a biobank. On the basis of the RNA samples, a cDNA sequence is obtained from mRNA by reverse transcription (RT-PCR) using specific primers of the human red opsin gene. Using the cDNA product obtained, the specific gene coding for the opsin protein of interest will be identified and amplified using polymerase chain reaction (PCR) to obtain sufficient cDNA for further processing. To ensure there are no mutations affecting the opsin sequence, a mutation screening is performed using next-generation sequencing (NGS) to compare the amplified gene against a reference sequence.

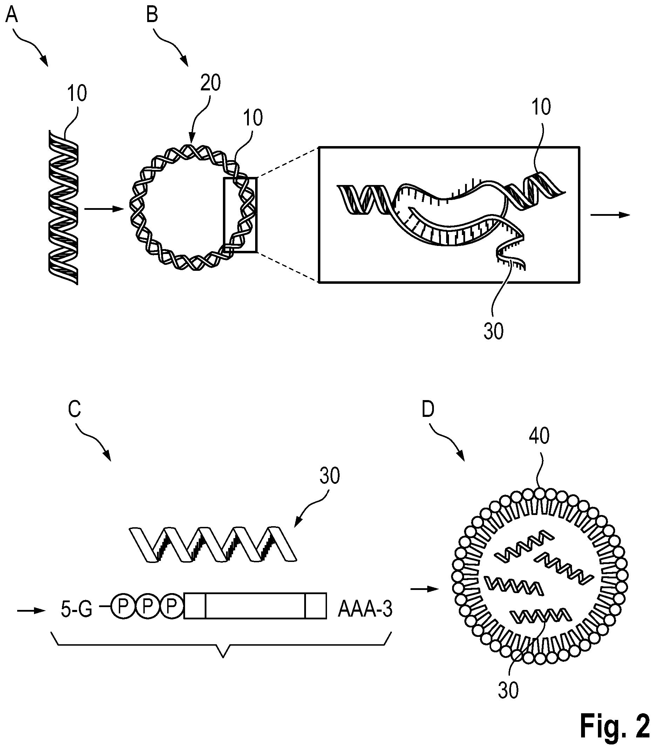

In a second step S2 the obtained cDNA 10 comprising an open reading frame encoding the amino acid sequence of the human red opsin protein is inserted into a cloning/expression vector (plasmid) 20 (see point A in Fig. 2). This vector is then introduced into host cells, which replicate to produce numerous copies of the cDNA. The cDNA contains all relevant sequences need for docking of a DNA-dependent RNA polymerase (for illustration of the transcription see point B in Fig. 2).

Following DNA sequence is used as the cDNA sequence for the human red opsin protein (Sequence number (ID): 1):

gccagggccg gctgccgtcg gggacagggc tttccatagc catggcccag cagtggagcc
tccaaaggct cgcaggccgc catccgcagg acagctatga ggacagcacc cagtccagca
tcttcaccta caccaacagc aactccacca gaggcccctt cgaaggcccg aattaccaca
tcgctcccag atgggtgtac cacctcacca gtgtctggat gatctttgtg gtcactgcat
ccgtcttcac aaatgggctt gtgctggcgg ccaccatgaa gttcaagaag ctgcgccacc
cgctgaactg gatcctggtg aacctggcgg tcgctgacct agcagagacc gtcatcgcca
gcactatcag cattgtgaac caggtctctg gctacttcgt gctgggccac cctatgtgtg
tcctggaggg ctacaccgtc tccctgtgtg ggatcacagg tctctggtct ctggccatca
tttcctggga gagatggatg gtggtctgca agccctttgg caatgtgaga tttgatgcca
agctggccat cgtgggcatt gccttctcct ggatctgggc tgctgtgtgg acagccccgc
ccatctttgg ttggagcagg tactggcccc acggcctgaa gacttcatgc ggcccagacg
tgttcagcgg cagctcgtac cccggggtgc agtcttacat gattgtcctc atggtcacct
gctgcatcat cccactcgct atcatcatgc tctgctacct ccaagtgtgg ctggccatcc
gagcggtggc aaagcagcag aaagagtctg aatccaccca gaaggcagag aaggaagtga
cgcgcatggt ggtggtgatg atctttgcgt actgcgtctg ctggggaccc tacaccttct
tcgcatgctt tgctgctgcc aaccctggtt acgccttcca ccctttgatg gctgccctgc
cggcctactt tgccaaaagt gccactatct acaaccccgt tatctatgtc tttatgaacc
ggcagtttcg aaactgcatc ttgcagcttt tcgggaagaa ggttgacgat ggctctgaac

tctccagcgc ctccaaaacg gaggtctcat ctgtgtcctc ggtatcgcct gcatgaggtc
tgcctcctac ccatcccgcc caccggggct ttggccacct ctcctttccc cctccttctc
catccctgta aaataaatgt aatttatctt tgccaaaacc aa

In an alternative approach, when in step S1 the blue opsin protein is selected, following DNA sequence is used as the cDNA sequence (Sequence number (ID): 2):

atccatgaga aaaatgtcgg aggaagagtt ttatctgttc aaaaatatct cttcagtggg
gccgtgggat gggcctcagt accacattgc ccctgtctgg gccttctacc tccaggcagc
tttcatgggc actgtcttcc ttatagggtt cccactcaat gccatggtgc tggtggccac
actgcgctac aaaaagttgc ggcagcccct caactacatt ctggtcaacg tgtccttcgg
aggcttcctc ctctgcatct tctctgtctt ccctgtcttc gtcgccagct gtaacggata
cttcgtcttc ggtcgccatg tttgtgcttt ggagggcttc ctgggcactg tagcaggtct
ggttacagga tggtcactgg ccttcctggc ctttgagcgc tacattgtca tctgtaagcc
cttcggcaac ttccgcttca gctccaagca tgcactgacg gtggtcctgg ctacctggac
cattggtatt ggcgtctcca tcccaccctt ctttggctgg agccggttca tccctgaggg
cctgcagtgt tcctgtggcc ctgactggta caccgtgggc accaaatacc gcagcgagtc
ctatacgtgg ttcctcttca tcttctgctt cattgtgcct ctctccctca tctgcttctc
ctacactcag ctgctgaggg ccctgaaagc tgttgcagct cagcagcagg agtcagctac
gacccagaag gctgaacggg aggtgagccg catggtggtt gtgatggtag gatccttctg
tgtctgctac gtgccctacg cggccttcgc catgtacatg gtcaacaacc gtaaccatgg
gctggactta cggcttgtca ccattccttc attcttctcc aagagtgctt gcatctacaa
tcccatcatc tactgcttca tgaataagca gttccaagct tgcatcatga agatggtgtg
tgggaaggcc atgacagatg aatccgacac atgcagctcc cagaaaacag aagtttctac
tgtctcgtct acccaagttg gccccaactg aggacccaat attggcctgt ttgcaacagc
tagaattaaa ttttactttt aagtaa

In a further alternative approach, when in step S1 the green opsin protein is selected, following DNA sequence is used as the cDNA sequence (Sequence number (ID): 3):

cccactggcc ggtataaagc accgtgaccc tcaggtgacg caccagggcc ggctgccgtc
ggggacaggg ctttccatag ccatggccca gcagtggagc ctccaaaggc tcgcaggccg

ccatccgcag gacagctatg aggacagcac ccagtccagc atcttcacct acaccaacag
caactccacc agaggcccct tcgaaggccc gaattaccac atcgctccca gatgggtgta
ccacctcacc agtgtctgga tgatctttgt ggtcattgca tccgtcttca caaatgggct
tgtgctggcg gccaccatga agttcaagaa gctgcgccac ccgctgaact ggatcctggt
gaacctggcg gtcgctgacc tggcagagac cgtcatcgcc agcactatca gcgttgtgaa
ccaggtctat ggctacttcg tgctgggcca ccctatgtgt gtcctggagg gctacaccgt
ctccctgtgt gggatcacag gtctctggtc tctggccatc atttcctggg agagatggat
ggtggtctgc aagccctttg gcaatgtgag atttgatgcc aagctggcca tcgtgggcat
tgccttctcc tggatctggg ctgctgtgtg gacagccccg cccatctttg gttggagcag
gtactggccc cacggcctga agacttcatg cggcccagac gtgttcagcg gcagctcgta
ccccggggtg cagtcttaca tgattgtcct catggtcacc tgctgcatca ccccactcag
catcatcgtg ctctgctacc tccaagtgtg gctggccatc cgagcggtgg caaagcagca
gaaagagtct gaatccaccc agaaggcaga gaaggaagtg acgcgcatgg tggtggtgat
ggtcctggca ttctgcttct gctggggacc atacgccttc ttcgcatgct ttgctgctgc
caaccctggc taccccttcc accctttgat ggctgccctg ccggccttct ttgccaaaag
tgccactatc tacaaccccg ttatctatgt ctttatgaac cggcagtttc gaaactgcat
cttgcagctt ttcgggaaga aggttgacga tggctctgaa ctctccagcg cctccaaaac
ggaggtctca tctgtgtcct cggtatcgcc tgcatgaggt ctgcctccta cccatcccgc
ccaccggggc tttggccacc tctcctttcc ccctccttct ccatccctgt aaaataaatg
taatttatct ttgccaaaac caacaaagtc acagaggctt tcactgcagt gtgggaccac
ctgagcctct gcgtgtgcag gcactgggtc tcgagagggt gcaaggggga taaagaggag
agagcgcttc atagacttta agttttcccg agcctcatgt ctaccgatgg cgtgaaagga
tcctggcaaa acagaagtgt gaggcaggtg ggcgtctata tccatttcac caggctggtg
gttacataat cggcaagcaa gagctgtgga ggggcttgct ggatgccctc agcacccagg
aggagggagg gagctagcaa gctaaggcag gtggccctcc tggcccctta aggtccatct
gctggaggcc cagagtcctt ggagtacagt ctacacctgg aggggaccca ttcctgccag
 tctgtggcag ggatggcgcg ccacctctgc caggccagga ccccaagccc gatcagcatc
agcatggtgc aggtgcacag gcgtgagctg atcagtgacg aggggcaggc acacaaggtg
gagacaaaga ccaagaggac ggttgccagt gagaggcgcg gactcaggaa cttgaacaac
atctgcgggg gacggctttg gaggtgctcc gctgcctcca gttgggtgac ttgctgtagc
atctccagct tggatattcg gctcttgaag gtctccgtga tctcctgcag gagacgaaaa
tgcacgcacc agaagtca

In a third step S3 mRNA (30) is produced by *in vitro* transcription. The plasmid 20 provided in step S2 contains the cDNA sequence which is used as a template. For *in vitro* transcription, a commercially available kit can be used which comprises all necessary components. Briefly, the *in vitro* transcription is carried out in a micro reaction tube. In an overall buffered aqueous reaction volume of 20 µl, 1 µg of DNA template are provided, 2 µl of a 100 mM solution of each nucleotide (ATP, GTP, UTP and CTP), and an RNA polymerase. The reaction volume is mixed, centrifuged and incubated at 37°C for 2h. The mature mRNA 30 transcribed from the cDNA sequence should contain these elements: a 5'cap, a 5'UTR, a coding region, a 3'UTR, and a 3' poly(A) tail (see point C of Fig. 2). These elements ensure that the mRNA will be properly processed, stable and efficiently translate into a protein.

The synthesized mRNA 30 is then purified. For purification, a commercially available kit can be used. Briefly, the volume of the above reaction is diluted with an appropriate buffer and then applied to a column with an RNA binding matrix. After at least two washing steps the mRNA 30 is eluted with water or an appropriate buffer.

In a fourth step S4 the purified RNA 30 is formulated with Lipid-nanoparticles (LNP) 40 (see point D in Fig. 2). The preparation of LNPs 40 is known to a person skilled in the art. Briefly, LNPs 40 comprise phospholipids, cholesterols, ionizable lipids, and polyethylene glycol-derived lipids. The synthesized mRNA 30 is rapidly mixed with the specified lipid components. To address a targeted biodistribution, the LNPs 40 can be coated with molecules, e.g. specific ligands which bind to receptors on the surface of targeted cone photoreceptor cells for recognition. A specific receptor to be used is the red opsin molecule, which is a transmembrane protein found in the outer segment of red cone photoreceptors. This molecule can be used as a ligand to specifically target these cells and minimizing off-target effects.

In a fifth step S5 the LNPs 40 are then formulated within a pharmaceutical solution. The solution is particularly suitable for application into an eye, namely into the vitreous cavity. Appropriate formulations are familiar to a person skilled in the art. An exemplary pharmaceutical formulation contains the LNPs 40 in a buffer with a pH in the range of 4 to 8, preferably 5 to 7 and is an aqueous solution containing sucrose, polysorbate, chlorides and phosphates.

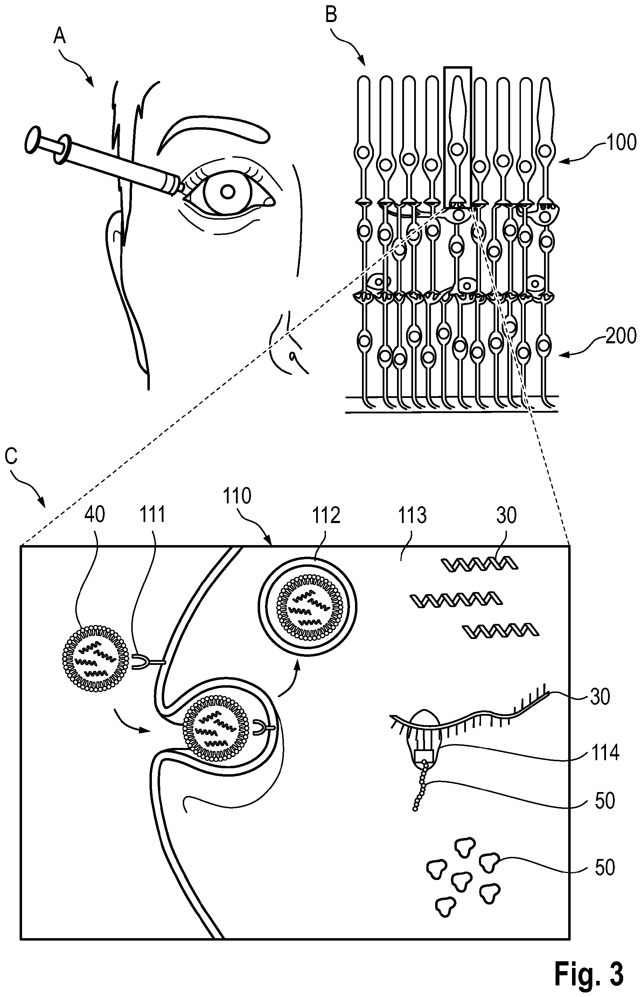

In a possible application of the pharmaceutical composition containing the synthetic mRNA 30 according to Fig. 3, it is delivered into the vitreous humor of the eye using a syringe (point A of Fig. 3). Within the eye, cone photoreceptor cells 100 are targeted (point B of Fig. 3). The LNPs 40 are taken up by the cone photoreceptor cells by endocytosis.

In point C of Fig. 3 a drawing of a single cone photoreceptor cell 110 is shown in order to illustrate the take-up of an LNP 40 by the cell 110 and further processes. The LNP 40 has surface molecules which bind to the molecule 111 which is expressed on the surface of the cell 110. The LNP 40 is fusing with the cell membrane of a cell 110 in a process called endocytosis. The LNP is 40 forming an endosome 112 which is moving in the cytoplasm 113 until release of the synthetic mRNA 30. The mRNA 30 is then recognized and bound by ribosomes 114, which produce the red opsin protein 50 (point 3 of Fig. 3). The amount of produced red opsin protein 50 can be controlled by the amount of mRNA 30 applied to the eye.

### Reference list

- 1: method
- S1: first step
- S2: second step
- S3: third step
- S4: fourth step
- S5: fifth step

- 10: DNA
- 20: Expression vector
- 30: mRNA
- 40: LNP
- 50: Red Opsin Protein
- 100: cone photoreceptor cells
- 110: single cone photoreceptor cell
- 111: surface marker
- 112: endosome
- 113: cytoplasm
- 114: ribosome
- 200: retinal neurons

## Claims

1. Synthetic mRNA molecule comprising a sequence encoding an opsin protein for use in a therapeutic method for preventing and/or treating myopia.

2. Synthetic mRNA molecule of claim 1, **characterized in that** the encoded opsin protein is at least one selected from the group comprising the human red opsin protein, the human green opsin protein, and the human blue opsin protein.

3. Synthetic mRNA molecule of claim 2, **characterized in that** the synthetic mRNA molecule comprises an open reading frame (ORF) comprising at least one of a nucleotide sequence having at least 90%, 95%, or 100% identity with the nucleotide sequence of the coding sequence of the human red opsin protein, the human blue opsin protein, or the human green opsin protein.

4. Use of a synthetic mRNA comprising a sequence comprising an open reading frame comprising at least one of a nucleotide sequence having at least 90%, 95%, or 100% identity with the nucleotide sequence of the coding sequence of the human red opsin protein, the human green opsin protein, or the human blue opsin protein for the manufacture of a pharmaceutical composition for treatment and/or prophylaxis of development of myopia.

5. Pharmaceutical formulation containing a synthetic mRNA comprising an ORF for an opsin protein together with at least one pharmaceutically acceptable carrier, cryoprotectant, lyoprotectant, excipient and/or diluent.

6. Pharmaceutical formulation of claim 5, **characterized in that** it contains at least one additional synthetic mRNA providing a coding information for at least one additional biomolecule selected from the group comprising scleral collagen, nyctalopin, glucagon, somatostatin, neurotensin, nitric oxide synthase enzymes, transforming growth factor betas and basic fibroblast growth factor.

7. Pharmaceutical formulation of claim 5 or 6, **characterized in that** the mRNA is formulated with at least one delivery agent selected from the group comprising lipid nanoparticles, lipids, polymers, peptides, virus-like particles, and cationic emulsion.

8. Pharmaceutical formulation of claim 7, **characterized in that** the mRNA is used for the preparation of lipid nanoparticles encapsulating the molecule.

9. Pharmaceutical formulation of claim 5, 6, 7, or 8, **characterized in that** it is suitable for intravitreal injection.

10. Synthetic mRNA molecule according to claims 1 to 4 or pharmaceutical formulation according to claims 5 to 9 for use as medicament.

11. Method for manufacturing a pharmaceutical formulation comprising a synthetic mRNA carrying the coding information for an opsin protein, the method comprising the steps of:
- Selecting a target opsin protein whose expression needs to be increased,
- Providing DNA encoding the amino sequence of the target opsin protein within an expression vector,
- Producing mRNA by *in vitro* transcription,
- Mixing the synthesized mRNA with at least one delivery agent,
- Formulating the mixture of the synthesized mRNA and the delivery agent into a pharmaceutical composition.

12. Method of claim 11, **characterized in that** the delivery agent is a lipid nanoparticle.

13. Method of claim 11 or 12, **characterized in that** the selected target protein is one selected from the group comprising the human red opsin protein as found in cones of the human eye.

14. Method of claim 11, 12 or 13, **characterized in that** the DNA template comprises an ORF with the sequence of the coding sequence of an opsin protein selected from the group comprising the human red opsin protein, the human green opsin protein, and the human blue opsin protein.
